(19) Europäisches Patentamt European Patent Office Office européen des brevets

(11) **EP 2 736 503 B1**

(12) **FASCICULE DE BREVET EUROPEEN**

(45) Date de publication et mention de la délivrance du brevet:
**08.07.2015 Bulletin 2015/28**

(21) Numéro de dépôt: **12740965.4**

(22) Date de dépôt: **27.07.2012**

(51) Int Cl.:
**A61K 31/165** (2006.01)     **A61P 9/00** (2006.01)
**A61P 9/10** (2006.01)

(86) Numéro de dépôt international:
**PCT/EP2012/064764**

(87) Numéro de publication internationale:
**WO 2013/014263 (31.01.2013 Gazette 2013/05)**

(54) **MEDICAMENT A BASE DE LEVOMILNACIPRAN POUR LA REHABILITATION FONCTIONNELLE APRES ACCIDENT NEUROLOGIQUE AIGU**

LEVOMILNACIPRAN-ARZNEIMITTEL ZUR FUNKTIONELLEN REHABILITIERUNG NACH EINEM AKUTEN NEUROLOGISCHEN SCHLAGANFALL

LEVOMILNACIPRAN DRUG FOR FUNCTIONAL REHABILITATION AFTER AN ACUTE NEUROLOGICAL STROKE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **28.07.2011 FR 1156917**

(43) Date de publication de la demande:
**04.06.2014 Bulletin 2014/23**

(73) Titulaire: **Pierre Fabre Médicament**
**92100 Boulogne-Billancourt (FR)**

(72) Inventeur: **SOKOLOFF, Pierre**
**F-81540 Belleserre (FR)**

(74) Mandataire: **Regimbeau**
**20, rue de Chazelles**
**75847 Paris Cedex 17 (FR)**

(56) Documents cités:
**EP-A1- 1 767 217     WO-A1-2011/088331**
**US-A1- 2004 162 334**

- **YAMAKAWA YURIKO ET AL: "Efficacy of milnacipran on poststroke depression on inpatient rehabilitation.", PSYCHIATRY AND CLINICAL NEUROSCIENCES DEC 2005 LNKD- PUBMED:16401247, vol. 59, no. 6, décembre 2005 (2005-12), pages 705-710, XP002669614, ISSN: 1323-1316**
- **SATO SHINJI ET AL: "Efficacy of milnacipran on cognitive dysfunction with post-stroke depression: preliminary open-label study.", PSYCHIATRY AND CLINICAL NEUROSCIENCES OCT 2006 LNKD- PUBMED:16958942, vol. 60, no. 5, octobre 2006 (2006-10), pages 584-589, XP002669615, ISSN: 1323-1316**
- **KIMURA M ET AL: "THERAPEUTIC EFFECTS OF MILNACIPRAN, A SEROTONIN AND NORADRENALINE REUPTAKE INHIBITOR, ON POST-STROKE DEPRESSION", INTERNATIONAL CLINICAL PSYCHOPHARMACOLOGY, CLINICAL NEUROSCIENCE PUBLISHERS, LONDON, GB, vol. 17, no. 3, 1 mai 2002 (2002-05-01), pages 121-125, XP008065711, ISSN: 0268-1315, DOI: 10.1097/00004850-200205000-00005**

**Description**

Il existe deux types d'accidents neurologiques aigus conduisant à des déficits moteurs et cognitifs : l'un est d'origine vasculaire, c'est l'Accident Vasculaire Cérébral, l'autre est d'origine traumatique, c'est le Traumatisme Crânien.

D'après l'OMS, l'Accident Vasculaire Cérébral (AVC) est "le développement rapide de signes cliniques localisés ou globaux de dysfonction cérébrale avec des symptômes durant plus de 24 heures, pouvant conduire à la mort, sans autre cause apparente qu'une origine vasculaire ". On parle aussi d'attaque cérébrale ou d'apoplexie. L'AVC est à distinguer de l'accident ischémique transitoire (AIT) défini comme "la perte brutale d'une fonction cérébrale ou oculaire durant moins de 24 heures supposée due à une embolie ou à une thrombose vasculaire". L'AVC est le type de maladies neurologiques le plus fréquent : dans les pays occidentaux il représente la troisième cause de décès (après les maladies coronariennes et les cancers) et la première cause de handicaps acquis à l'âge adulte et la deuxième de démence (Murray CJ, Lopez AD, Mortality by cause for eight régions of the world: Global Burden of Disease Study, Lancet, 1997;349:1269-1276).

Dans l'AVC, le problème vasculaire en cause est soit d'ordre thrombo-embolique (80% des AVC), dû à l'arrêt de la circulation sanguine par obstruction d'une artère, soit d'ordre hémorragique (20% des AVC), par rupture d'une artère. La thrombose cérébrale est le plus souvent causée par l'artériosclérose (durcissement et inflammation de la paroi vasculaire). L'arrêt de circulation secondaire à la thrombose artérielle (obstruction par un caillot sanguin) est à l'origine d'un infarctus (mort, nécrose de la zone touchée) accompagné d'un ramollissement du territoire correspondant et qui n'est plus irrigué. Progressivement, le tissu mort est remplacé par du tissu conjonctif formé de cellules gliales. Une autre cause d'infarctus est l'embolie cérébrale, où une plaque d'athérome (corps gras) peut se détacher d'un gros vaisseau, ou bien encore lorsque qu'un caillot sanguin est formé, par exemple dans les cardiopathies emboligènes (infarctus du myocarde, valvulopathies, arythmie par fibrillation auriculaire), et vient obstruer une artère cérébrale et provoquer un infarctus. L'hémorragie cérébrale peut aussi être due à l'artériosclérose, le plus souvent accompagnée d'hypertension artérielle. Les hémorragies cérébrales peuvent également être occasionnées par une malformation congénitale artérielle, une infection, une tumeur cérébrale, ou même une contrariété, une émotion ou un effort violent. L'hémorragie est à l'origine de la formation d'un hématome qui sera résorbé progressivement.

Le diagnostic de l'AVC est d'abord clinique. L'examen des capacités motrices et de la sensibilité de tout ou partie du corps orientent vers le siège des lésions, qui est confirmé par imagerie cérébrale. Le diagnostic peut être problématique pour les patients comateux, aphasiques ou amnésiques. Les symptômes cliniques sont de gravité qui varie depuis l'absence de signe remarquable jusqu'à la mort dans un délai de quelques jours, et peuvent inclure des troubles de la motricité, de la coordination et de la marche, de la sensibilité, de la parole, du champ visuel, de la mémoire et du psychisme.

Le traitement de l'AVC est instauré immédiatement après l'événement et prend en compte l'origine ischémique ou hémorragique, déterminée par imagerie cérébrale en utilisant le scanner, le scanner cérébral sans injection et l'imagerie par résonance magnétique (IRM). Pour les AVC ischémiques, le traitement vise à équilibrer la balance hydroélectrolytique et la pression artérielle et à obtenir la reperfusion du territoire atteint par des agents thrombolithiques, comme les antiplaquettaires (comme l'aspirine) et les fibrinolytiques (par exemple, rt-PA, pour recombinant tissue plasminogen activator) lorsque l'AVC est pris en charge moins de 4 h 30 après les premiers signes. Pour les AVC hémorragiques, l'intervention chirurgicale est indiquée lorsqu'elle est possible en fonction de la topographie et du volume de l'hématome, du niveau de conscience du patient et de son état général. La récupération, après la phase aiguë, est très progressive et peut s'étaler sur plusieurs mois ou années. Elle nécessite souvent une rééducation afin de faire régresser les troubles de la parole et/ou de la marche. Si les troubles moteurs (des mouvements) et sensitifs (des sensations) sont généralement récupérables, les séquelles intellectuelles peuvent être irréversibles.

Les traumatismes crâniens (TC) sont la principale cause de mortalité et de handicap sévère avant 45 ans. Les causes principales sont : les accidents de la voie publique (environ 50 %), les accidents sportifs, les accidents du travail, les accidents domestiques, les agressions, les catastrophes naturelles et les faits de guerre. Il existe différents types de TC :

- la commotion cérébrale : il s'agit d'un ébranlement du cerveau consécutif à un choc violent sur le crâne, accompagné ou non d'une perte de connaissance temporaire ou initiale, sans lésion visible radiologiquement dans le cerveau. Le réveil survient spontanément quelques secondes, minutes ou heures après le traumatisme en fonction de l'importance du choc, et peut laisser des troubles transitoires de la mémoire, voire des complications secondaires : hématome extra-dural, hématome sous-dural, oedème cérébral.
- la contusion cérébrale : dans ce cas, il existe des lésions anatomiques du cerveau (nécrose hémorragique avec oedème), pas nécessairement au niveau de l'impact, qui peuvent se compliquer d'un oedème cérébral.
- le coma profond d'emblée : c'est une commotion de gravité maximale. Le patient présente un coma profond et persistant après le choc car le dysfonctionnement de la substance réticulée ascendante est plus profond. Des signes de décérébration sont possibles témoignant de la présence de lésions mésencéphaliques et axonales diffuses liés à la propagation concentrique et à la concentration des ondes de choc vers le centre du cerveau (phénomènes stéréotaxiques).

La prise en charge des TC comprend la recherche par imagerie cérébrale de lésions curables chirurgicalement (hématome), l'intervention sur une lésion opérable, ou, dans le cas contraire, un traitement médical de réanimation est entrepris en milieu spécialisé (anti-oedémateux, réanimation respiratoire etc.). On utilise des diurétiques pour diminuer l'oedème cérébral, et du mannitol, qui permet de déshydrater le tissu cérébral. Parfois, l'oedème cérébral est assez important pour provoquer un début d'engagement cérébral (engagement de la partie basse du cerveau sous la faux du cerveau vers l'hémisphère cérébral controlatéral, engagement de la partie inférieure du cerveau dans le trou occipital). Une hémorragie méningée peut être associée à une contusion cérébrale, et se traduit par des maux de tête, une raideur de nuque et des troubles de la conscience. Une surveillance clinique et radiologique de l'évolution succède au traitement d'urgence. Le pronostic dépend de l'importance de lésions initiales, de l'âge et de l'état général du patient avant l'accident. Plus le coma est superficiel et le patient jeune et en bonne santé avant l'accident, plus les chances de guérison sont grandes. Mais le coma peut entraîner une mort cérébrale dans certains cas.

Après la période critique suivant le traumatisme et la reprise de conscience, se met en place, comme dans le cas des AVC, une période de récupération fonctionnelle qui peut laisser des séquelles neurologiques : des signes plégiques ou paralytiques, des troubles de l'équilibre, des troubles symboliques, type aphasiques ou agnosiques, des signes de lésions des nerfs crâniens ; des troubles neuroendocriniens : diabète insipide, perte de poids, fatigue, étourdissements, perte de libido, et impuissance ; et des troubles psychiques : angoisse après la prise de conscience de séquelles potentiellement irrémédiables, anhédonie. D'autres conséquences sont moins fréquentes : épilepsies post-traumatiques ultérieures, troubles vasculaires tels que des ruptures d'anévrismes ou des thromboses d'artères à destination cérébrale.

Le domaine de l'invention se situe dans l'intervention médicamenteuse qui vise à améliorer la récupération et la réhabilitation fonctionnelle après accident neurologique aigu, qu'il soit un AVC ou un TC. Dans le contexte de l'invention, améliorer la récupération et la réhabilitation fonctionnelle signifie accélérer et amplifier la régression des symptômes moteurs, neurophysiologiques, cognitifs ou psychiatriques apparus lors de l'accident neurologique, ou bien d'un ou plusieurs de ces symptômes. Selon l'invention, les symptômes moteurs, neurophysiologiques, cognitifs et psychiatriques incluent, de manière non exhaustive :

- les paralysies et plégies, dont hémiplégies et tétraplégies,
- les paresthésies ou troubles de la sensibilité,
- les troubles de la coordination, ataxie des extrémités et à la marche,
- les troubles de la motricité oculaire,
- les troubles de la déglutition,
- les troubles de la parole, qu'ils concernent la perception et la compréhension, ou l'expression,
- les apraxies et les perturbations de la notion de l'espace,
- les troubles de la vue et du champ visuel,
- les anomalies des pupilles,
- les troubles de l'attention,
- les troubles de la mémoire, touchant la mémoire à court terme (faits récents) ou la mémoire à long terme (faits anciens),
- les troubles perceptifs, essentiellement visuels, concernent la reconnaissance d'objets, d'images, de l'écriture ou des physionomies,
- les troubles des fonctions exécutives, telles que la planification des actions,
- l'angoisse,
- l'anhédonie et les symptômes dépressifs,
- la persévération,
- l'impulsivité.

L'invention concerne aussi la récupération et la réhabilitation fonctionnelle après un accident neurologique survenant comme récidive à un accident neurologique initial, provoqué par ses conséquences sur l'équilibre postural, la perte de vue ou la négligence visiospatiale.

Des études chez l'animal et l'Homme démontrent la possibilité d'accélérer ou d'amplifier la récupération fonctionnelle après un accident neurologique aigu, par l'administration d'un traitement médicamenteux immédiatement ou même après un délai de quelques jours à quelques mois suivant l'accident. Dans les modèles animaux d'occlusion unilatérale de l'artère cérébrale médiane, qui miment l'AVC, et les modèles de lésion focale du cortex, qui miment le TC (Goldstein LB. Basic and clinical studies of pharmacological effects on recovery from brain injury. J neural Transplant & Plasticity, 1993, 4: 175-192; Feeney DM, de Smet AM, Rai S, Noradrenergic modulation of hemiplegia: facilitation and maintenance of recovery. Restor Neurol & Neurosci, 2004, 22 :175-190), les traitements médicamenteux qui se sont révélés actifs chez le rat et le chat, sont :

- l'amphétamine, un produit qui augmente les taux extracellulaire de noradrénaline, sérotonine et dopamine
- la transplantation de cellules chromaffines, qui secrètent de la noradrénaline
- l'infusion intracérébrale de noradrénaline, mais pas de sérotonine ou de dopamine
- l'administration d'un précurseur de noradrénaline
- l'administration d'agonistes alpha-adrénergiques.

Les études cliniques, sur des petits groupes de patients, ont montré la possibilité d'améliorer la récupération motrice après AVC lors d'un traitement avec l'amphétamine (Sonde L, Nordström M, Nilsson CG, Lökk J, Viitanen M, A double-blind placebo-controlled study of the effects of amphetamine and physiotherapy after stroke. Cerebrovasc Dis, 2001;12:253-257) ; le L-DOPS, un précurseur de noradrénaline (Nishino K, Sasaki T, Takahashi K, Chiba M, Ito T, The norepinephrine precursor L-threo-3,4-dihydroxyphenylserine facilitates motor recovery in chronic stroke patients. J Clin Neurosci, 2001, 8:547-550), le methylphénidate, un agent qui augmente aussi les taux extracellulaire de noradrénaline, sérotonine et dopamine (Tardy J, Pariente J, Leger A, Dechaumont-Palacin S, Gerdelat A, Guiraud V, Conchou F, Albucher JF, Marque P, Franceries X, Cognard C, Rascol O, Chollet F, Loubinoux I, Methylphenidate modulates cerebral post-stroke reorganization. Neuroimage, 2006, 33:913-922), la réboxétine, un inhibiteur sélectif de recapture de la noradrénaline (Zittel S, Weiller C, Liepert J, Reboxetine improves motor function in chronic stroke. A pilot study. J Neurol, 2007, 254:197-201), la fluoxétine, un inhibiteur sélectif de recapture de la sérotonine (Pariente J, Loubinoux I, Carel C, Albucher JF, Leger A, Manelfe C, Rascol O, Chollet F, Fluoxetine modulates motor performance and cerebral activation of patients recovering from stroke. Ann Neurol, 2001, 50:718-729). L'effet de la fluoxétine administrée pendant 3 mois a été confirmé dans une plus large étude clinique en double insu contre placebo (Chollet F, Tardy J, Albucher JF, Thalamas C, Berard E, Lamy C, Bejot Y, Deltour S, Jaillard A, Niclot P, Guillon B, Moulin T, Marque P, Pariente J, Arnaud C, Loubinoux I, Fluoxetine for motor recovery after acute ischaemic stroke (FLAME): a randomised placebo-controlled trial. Lancet Neurol, 2011, 10:123-30).

Il est important de souligner que ces approches thérapeutiques ne visent pas à restaurer ou à protéger la zone cérébrale lésée, mais à permettre au cerveau, qui jouit d'une certaine plasticité, à réorganiser ses circuits pour permettre à des zones non lésées d'assurer les fonctions normalement dévolues à la zone lésée, qu'elles soient motrices, neurophysiologiques ou cognitives. Ceci a été confirmé en imagerie fonctionnelle dans l'AVC et le TC. La faculté des monoamines (noradrénaline, sérotonine, dopamine) à favoriser la réorganisation fonctionnelle du cerveau est en accord avec leur rôle neurotrophique connu dans le développement pour assurer la différenciation et la survie des neurones.

De ces données montrant le rôle crucial de la sérotonine et de la noradrénaline, dans la récupération fonctionnelle, il est conclu qu'un médicament qui produit une élévation des taux extracellulaires à la fois de la noradrénaline et de la sérotonine présenterait un avantage par rapport aux médicaments qui n'augmentent que la sérotonine, comme la fluoxétine, pour le traitement des suites d'un accident neurologique aigu.

Le lévomilnacipran est l'énantiomère (*1S, 2R*) du milnacipran (Z (±)-2-aminométhyl)-N,N'-diéthyl-1-phényl cyclopropane carboxamide) décrit dans les brevets WO 2004/075886 et WO 2009/127737. Le milnacipran est un inhibiteur de la recapture de noradrénaline et de sérotonine, avec un effet équilibré sur ces deux neuromédiateurs (Briley M, Prost JF, Moret C, Preclinical pharmacology of milnacipran. Int Clin Psychopharmacol, 1996 Suppl 4:9-14 ; Preskorn SH, Milnacipran: a dual norepinephrine and serotonin reuptake pump inhibitor. J Psychiatr Pract, 2004, 10:119-26). Le milnacipran est un médicament utilisé dans la dépression (Spencer CM, and Wilde MI, Milnacipran : a review of its use in depression. Drugs, 1998, 56: 405-427) et la fibromyalgie (Owen RT, Milnacipran hydrochloride: its efficacy, safety and tolerability profile in fibromyalgia syndrome. Drugs Today (Barc), 2008, 44:653-60). Les demandes de brevets WO2003/039598, WO2003/068211, WO2003/077897, WO2003/090743, WO2004/009069, WO2004/030633, WO2004/045718, WO2007/038620, WO2008/019388, WO2008/021932 et WO2008/147843 décrivent aussi l'utilisation du milnacipran et de ses énantiomères dans le syndrome de fatigue chronique, le déficit attentionnel avec hyperactivité, les syndromes de la douleur viscérale, les syndromes somatiques fonctionnels, les troubles cognitifs et du sommeil, le syndrome du côlon irritable, les douleurs chroniques du bas du dos, les douleurs pelviennes chroniques, la cystite interstitielle, la douleur thoracique non cardiaque, la douleur neuropathique, le trouble de l'articulation temporo-mandibulaire, les douleurs faciales atypiques, les céphalées de tension, les sensibilités chimiques multiples, la douleur chronique associée à un traitement médicamenteux ou la radiothérapie ou d'autres indications de douleurs chroniques ; notablement ces demandes de brevets ne décrivent pas l'utilisation du milnacipran dans le traitement des AVC et TC.

Le lévomilnacipran est l'isomère réputé actif du milnacipran ; il a l'affinité la plus élevée pour les transporteurs de la noradrénaline et de la sérotonine, comparée à celle de l'autre énantiomère, le dextromilnacipran et bloque la recapture de noradrénaline et de sérotonine à des concentrations inférieures à celles nécessaires de dextromilnacipran (Exemple 1). Mais de manière surprenante, le dextromilnacipran est l'isomère le plus puissant sur le récepteur alpha1 adrénergique de rat ou humain (Exemple 1). De plus, le dextromilnacipran se comporte comme un antagoniste alpha1 :

il n'active pas le récepteur alpha1 recombinant humain et antagonise l'effet de l'adrénaline (Exemple 2).

Les données précliniques et cliniques indiquent que le récepteur alpha1 adrénergique joue un rôle crucial dans la récupération fonctionnelle après accident neurologique. En effet, une administration unique de prazosine, un antagoniste sélectif du récepteur alpha1 (Hoffman et Lefkowitz. Catecholamines, sympathomimetic drugs and adrenergic receptor antagonists. in Goodman and Gilman's The Pharmacological Basis of Therapeutics, Hardman JG, Limbird LE, Molinoff P B, Ruddon RW éditeurs, 9ème édition, 1995, McGraw-Hill New York, pp229) retarde la récupération fonctionnelle après une contusion traumatique unilatérale focale du cortex sensorimoteur chez le rat (Feeney DM, and Westerberg VS, Norepinephrine and brain damage: alpha noradrenergic pharmacology alters functional recovery after cortical trauma. Can J Psychology, 1990, 44: 233-252) et précipite la réapparition des symptômes moteurs chez le rat jusqu'à 6 mois après une lésion frontale unilatérale chez le rat, lorsque la récupération fonctionnelle motrice a été effective (Stibick DL, and Fennec DM, Enduring vulnerability to transient reinstatement of hemiplegia by prazosin after traumatic brain injury. J Neurotrauma, 2001, 18:303-312). Chez le volontaire sain, l'administration de prazosine décroit l'efficacité d'un entraînement à une tâche motrice pour induire une plasticité cérébrale dans le cortex, et ceci en l'absence de modification de l'excitabilité cortico-motrice (Sawaki L, Werhahn KJ, Barco R, Kopylev L, Cohen LG, Effect of an alpha1-adrenergic blocker on plasticity elicited by motor training. Exp Brain Res, 2003, 148:504-508). La plasticité induite par l'entraînement est supposée contribuer à la récupération fonctionnelle motrice après un accident neurologique aigu. Goldstein et al (The influence of drugs on the recovery of sensorimotor function after stroke. J Neuro Rehab, 1990, 4:137-144) ont réalisé une étude chez des patients victimes d'un AVC et ont constaté que ceux à qui étaient prescrits des médicaments ayant des effets délétères sur la récupération fonctionnelle chez les animaux expérimentaux, parmi lesquels figurait notablement la prazosine, avaient des scores moteurs sur l'échelle de Fugl-Meyer plus faibles que ceux ne recevant pas ces médicaments aux effets délétères, lors d'une évaluation prospective sur 30 jours suivant l'inclusion. L'ensemble de ces données précliniques et cliniques montre qu'il ne faut pas administrer un antagoniste alpha1 adrénergique à un patient en phase de récupération fonctionnelle après un accident neurologique aigu.

Il a donc été découvert de manière surprenante qu'il serait contre-indiqué d'administrer du dextromilnacipran, qui a été découvert comme un antagoniste alpha1 adrénergique dans l'élaboration de l'invention, à un patient dans la récupération fonctionnelle après accident neurologique aigu, qu'il soit un AVC ou un TC. Ainsi, contrairement à ce qui est prévu dans la demande WO2006/006617, le racémate milnacipran qui contient une proportion égale de lévomilnacipran et de dextromilnacipran, ne doit pas être prescrit dans les situations cliniques susmentionnées. Au contraire le lévomilnacipran substantiellement pur, ou bien un mélange lévomilnacipran / dextromilnacipran contenant du dextromilnacipran dans une proportion ne dépassant pas 5 % en masse dudit mélange (voir Exemple 3) doit être utilisé dans la récupération fonctionnelle après accident neurologique aigu, qu'il soit un AVC ou un TC.

Le brevet WO2004/075886 revendique l'utilisation du lévomilnacipran pour la préparation d'un médicament dans une variété de pathologies chez des patients présentant des risques cardiovasculaires, sur la base de l'observation que le lévomilnacipran induit moins de phénomènes hémodynamiques que le racémate milnacipran chez le chien. Cependant, ce brevet ne dévoile pas l'activité particulière du dextromilnacipran sur le récepteur alpha1 adrénergique et encore moins l'utilisation du lévomilnacipran dans la récupération fonctionnelle après AVC ou TC.

Encore selon l'invention, le lévomilnacipran est utilisé sous la forme d'un sel pharmaceutiquement acceptable, choisi parmi les sels inorganiques d'addition d'acides non-toxiques pour le patient auquel ils sont administrés. Le terme « pharmaceutiquement acceptable » se réfère à des entités moléculaires et des compositions qui ne produisent aucun effet adverse, allergique ou autre réaction indésirable quand elles sont administrées à un animal ou un humain. Des exemples de sels d'addition d'acide pharmaceutiquement acceptables incluent le bromhydrate, le chlorhydrate, le sulfate, le bisulfate, le phosphate, le nitrate, l'acétate, l'oxalate, le valérate, l'oléate, le palmitate, le stéarate, le laurate, le borate, le benzoate, le lactate, le phosphate, le tosylate, le citrate, le maléate, le fumarate, le succinate, le tartrate, les sels de naphthylate, et les semblables. (Voir, par exemple Berge SM, Bighley LD, Monkhouse DC, Pharmaceutical salts, 1977, 66:1-19). Le sel préféré dans le cadre de la présente invention est toutefois le chlorhydrate de lévomilnacipran.

L'invention concerne également une composition pharmaceutique caractérisée en ce qu'elle contient le lévomilnacipran à titre de principe actif et au moins un excipient pharmaceutiquement acceptable. Quand utilisé ici, le terme excipient pharmaceutiquement acceptable inclut tout diluant, adjuvant ou excipient, tels que des agents préservatifs, des agents de remplissage, des agents désintégrant, mouillant, émulsifiant, dispersant, antibactérien ou antifongique, ou bien encore des agents qui permettent de retarder l'absorption et la résorption intestinale et digestive. L'utilisation de ces milieux ou vecteurs est bien connue de l'homme du métier.

Les compositions pharmaceutiques peuvent contenir du lévomilnacipran substantiellement pur, ou bien des mélanges de lévomilnacipran et de dextromilnacipran, à condition que la proportion de dextromilnacipran soit insuffisante pour que l'activité antagoniste alpha1 adrénergique soit significative et que le patient soit exposé à un blocage du récepteur alpha1 adrénergique. Une simulation de l'activité des mélanges lévomilnacipran/dextromilnacipran, confirmée par les données expérimentales, montre que l'activité anti-alpha1 devient significative pour des mélanges contenant plus de 5% de dextromilnacipran (Exemple 3). La proportion de dextromilnacipran dans un mélange lévomilnacipran/ dextromilnacipran ne doit donc pas excéder 5 % en masse dudit mélange.

Les compositions pharmaceutiques selon la présente invention peuvent être formulées pour l'administration aux mammifères, y compris l'homme. Les compositions selon l'invention peuvent être administrées par voie orale, sublinguale, sous-cutanée, intramusculaire, intraveineuse, transdermique, locale ou rectale. Dans ce cas l'ingrédient actif peut être administré sous formes unitaires d'administration, en mélange avec des supports pharmaceutiques classiques, aux animaux ou aux êtres humains. Les formes unitaires d'administration appropriées comprennent les formes par voie orale telles que les comprimés, les gélules, les poudres, les granules, chacune contenant une quantité prédéterminée de lévomilnacipran, elles incluent également les solutions ou suspensions orales dans un liquide aqueux ou un liquide non-aqueux, ou une émulsion liquide huile/eau ou eau/huile, les formes d'administration sublinguale et buccale, les formes d'administration sous-cutanée ou transdermique, topique, intramusculaire, intraveineuse, intra-nasale ou intraoculaires, et les formes d'administration rectale. Lorsque l'on prépare une composition solide sous forme de comprimés, on mélange le lévomilnacipran avec un véhicule pharmaceutique tel que la gélatine, l'amidon, le lactose, le stéarate de magnésium, le talc, la gomme arabique, la silice ou analogues. On peut enrober les comprimés de saccharose ou d'autres matières appropriées.

La libération dudit principe actif peut être retardée pour obtenir une libération prolongée de façon à permettre l'administration d'une seule dose quotidienne. Une telle formulation galénique peut être obtenue selon le procédé décrit dans le brevet EP 939 626 ou tout autre procédé.

On obtient une préparation en gélules en mélangeant l'ingrédient actif avec un diluant et en versant le mélange obtenu dans des gélules molles ou dures.

Une préparation sous forme de sirop ou d'élixir peut contenir l'ingrédient actif conjointement avec un édulcorant, un antiseptique, ainsi qu'un agent donnant du goût et un colorant approprié.

Les poudres ou les granules dispersibles dans l'eau peuvent contenir l'ingrédient actif en mélange avec des agents de dispersion ou des agents mouillant, ou des agents de mise en suspension, de même qu'avec des correcteurs du goût ou des édulcorants.

Pour une administration rectale, on recourt à des suppositoires qui sont préparés avec des liants fondant à la température rectale, par exemple du beurre de cacao ou des polyéthylènes glycols.

Pour une administration parentérale (intraveineuse, intramusculaire, intradermique, sous-cutanée), intra-nasale ou intraoculaire, on utilise des suspensions aqueuses, des solutions salines isotoniques ou des solutions stériles et injectables qui contiennent des agents de dispersion et/ou des agents mouillants pharmacologiquement compatibles.

Le principe actif peut également être formulé sous forme de microcapsules, éventuellement avec un ou plusieurs supports additifs.

Avantageusement, la composition pharmaceutique selon la présente invention est destinée à une administration par voie orale.

Les dosages des compositions pharmaceutiques contenant le lévomilnacipran dans les compositions de l'invention sont ajustés afin d'obtenir une quantité de substance active qui est efficace pour obtenir la réponse thérapeutique désirée pour une composition particulière à la méthode d'administration. Le niveau choisi de dosage dépend donc de l'effet thérapeutique désiré, de la voie de l'administration choisie, de la durée désirée du traitement, le poids, l'âge et le sexe du patient, la sensibilité de l'individu à traiter. En conséquence, la posologie optimale devra être déterminée en fonction des paramètres jugés pertinents, par le spécialiste en la matière.

Préférentiellement, le lévomilnacipran est administré dans des compositions pharmaceutiquement acceptables où la dose quotidienne de lévomilnacipran, exprimée en base, est comprise entre 25 et 200 mg, prise en une seule ou plusieurs fois par jour. Encore préférentiellement, la composition pharmaceutique permet une absorption intestinale modifiée de telle sorte qu'une seule prise par jour est suffisante.

Exemple 1

Mesure de l'affinité des deux isomères du milnacipran pour les transporteurs de la noradrénaline et de la sérotonine et pour le récepteur adrénergique alpha1

Les affinités du lévomilnacipran et du dextromilnacipran ont été mesurées sur la liaison aux transporteurs humains recombinants de la noradrénaline et de la sérotonine et sur la liaison au récepteur alpha1 humain recombinant. L'inhibition par ces deux produits pour la recapture de noradrénaline [³H] et de sérotonine [³H] a aussi été mesurée.

Méthodes :

-    Liaison au transporteur de la noradrénaline : la liaison a été mesurée sur des membranes de cellules MDCK exprimant ce transporteur, provenant de Perkin-Elmer (batch N° 418-165-A), diluées dans du tampon TRIS-HCl 50 mM, contenant du NaCl 120 mM et du KCl 5 mM, à la concentration de 5 $\mu$g de protéines, en présence de 2 nM de N-

methyl-nisoxetine [3H] et de concentrations croissantes de lévomilnacipran ou de dextromilnacipran ($10^{-11}$ à $10^{-5}$ M). La fraction liée a été séparée par filtration et lavage dans du tampon TRIS + NaCl + KCl refroidi. La liaison non spécifique a été mesurée en présence de 10 μM de désipramine.

- Liaison au transporteur de la sérotonine : la liaison a été mesurée sur des membranes de cellules MDCK exprimant ce transporteur, provenant de Perkin-Elmer (batch N° 316-199-A), diluées dans du tampon TRIS-HCl 50 mM, contenant du NaCl 120 mM et du KCl 5 mM, à la concentration de 5 μg de protéines, en présence de 2 nM de citalopram[3H] et de concentrations croissantes de lévomilnacipran ou de dextromilnacipran ($10^{-11}$ à $10^{-5}$ M). La fraction liée a été séparée par filtration et lavage dans du tampon TRIS + NaCl + KCl refroidi. La liaison non spécifique a été mesurée en présence de 10 μM de fluoxétine.

- Liaison au récepteur alpha1 humain recombinant : 1a liaison a été mesurée sur des membranes de cellules CHO (Chinese Hamster Ovary) exprimant le récepteur alpha1B humain (Wurch T, Boutet-Robinet EA, Palmier C, Colpaert FC, Pauwels PJ, Constitutive coupling of chimeric dopamine D2/alpha1B receptor to the phospholipase C pathway : inverse agonism to silent antagonism of neuroleptic drugs. J Pharmacol Exp Ther, 2003, 304 :380-390) diluées dans un tampon TRIS -HCl 50 mM à la concentration de 7.8 μg de protéines, en présence de 0.1 nM de prazosine[3H] et de concentrations croissantes de lévomilnacipran ou de dextromilnacipran ($10^{-11}$ à $10^{-5}$ M). La fraction liée , a été séparée par filtration et lavage dans du tampon TRIS refroidi. La liaison non spécifique a été mesurée en présence de 10 μM de phentolamine.

- Recapture de noradrénaline[3H] : des cellules CHO-K1 ont été transfectées de façon permanente par le gène du transporteur humain de la noradrénaline par impulsion électrique (Biorad gene pulser) et les clones transfectés ont ensuite été sélectionnés par incubation dans la généticine. Pour la mesure de recapture, les cellules transfectées ont été cultivées en plaques de 24 puits puis incubées en présence de pargyline et d'ascorbate (100 μM) et de noradrénaline[3H] (activité spécifique = 40,7 Ci/mole) à la concentration de 10 nM. La recapture a été stoppée par aspiration et rinçage du milieu et la radioactivité captée par les cellules comptée par scintillation liquide. Le signal non spécifique a été déterminé en présence 10μM de désipramine

- Recapture de sérotonine [3H] : des cellules CHO-K1 ont été transfectées de façon permanente par le gène du transporteur humain de la sérotonine par impulsion électrique (Biorad gene pulser) et les clones transfectés ont ensuite été sélectionnés par incubation dans la généticine. Pour la mesure de recapture, les cellules transfectées ont été cultivées en plaques de 24 puits puis incubées en présence de pargyline et d'ascorbate (100 μM) et de sérotonine[3H] (activité spécifique = 32,7 Ci/mole) à la concentration de 10 nM. La recapture a été stoppée par aspiration et rinçage du milieu et la radioactivité captée par les cellules comptée par scintillation liquide. Le signal non spécifique a été déterminé en présence 10 μM de fluoxétine.

Résultats :

Le tableau 1 indique les valeurs de constantes d'inhibition ($K_i$) du lévomilnacipran et dextromilnacipran pour les transporteurs de la sérotonine et de la noradrénaline, et du récepteur alpha1 adrénergique.

Tableau 1

| Cible | Valeur $K_i$ pour la liaison ou $IC_{50}$ pour la recapture (μM) | |
|---|---|---|
| | lévomilnacipran | dextromilnacipran |
| Liaison au transporteur noradrénaline (NET) | 0,091 | 10,5 |
| Liaison au transporteur sérotonine (SERT) | 0,011 | 0,32 |
| Inhibition de la recapture de noradrénaline [3H] | 0.010 | 0.15 |
| Inhibition de la recapture de sérotonine[3H] | 0.018 | 0.28 |
| Récepteur adrénergique α1A | 110 | 3,4 |

Exemple 2

Mesure de l'activité intrinsèque du dextromilnacipran aux récepteurs alpha1A et alpha1B recombinants humains
L'activité intrinsèque fonctionnelle du dextromilnacipran a été mesurée sur des cellules exprimant les récepteurs alpha1A et alpha1B humains pour déterminer son pouvoir agoniste/antagoniste.

Méthodes :

Des cellules CHO-K1 exprimant de manière stable le récepteur alpha1A humain ou le récepteur alpha1B humain ont été obtenues selon la méthode décrite (Vicentic et al. Biochemistry and pharmacology of epitope-tagged alpha1a-adrenergic receptor subtype. J Pharm Exp Ther, 2002, 302-58-65). L'activité agoniste a été évaluée par mesure en fluorimetry de la concentration intracellulaire de calcium, selon une technique conventionnelle utilisant un chélateur fluorescent de calcium et un enregistrement du signal par la technique dite du Fluorometric Imaging Plate Reader (FLIPR, Molecular Devices, Saint-Grégoire, France). La (-)adrénaline a servi de référence positive, les réponses ont ensuite été normalisées par rapport à celle de la (-)adrénaline à la concentration de 10 $\mu$M.

Résultats :

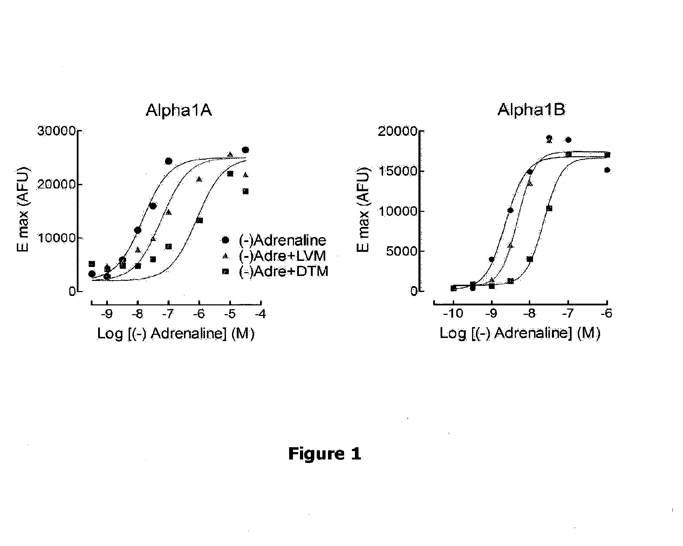

Dans une gamme de concentrations de 3. $10^{-7}$ M à $10^{-3}$ M, le dextromilnacipran n'a pas montré d'activité agoniste supérieure à 10% de l'activité de la (-)adrénaline, que ce soit sur le récepteur alpha1A ou alpha1B. La (-) adrénaline a été ensuite incubée en concentrations croissantes ($3.10^{-10}$ à $3.10^{-5}$ M) en présence de lévomilnacipran ou de dextromilnacipr a n à la concentration de 300 $\mu$M. La figure 1 montre que la courbe concentration-réponse de la (-) adrénaline est déplacée vers la droite par le dextromilnacipran d'un facteur d'environ 100 pour le sous-type alpha1A et d'environ 10 pour le sous-type alpha1B. Pour le lévomilnacipran, le déplacement n'est que d'environ 3 pour le sous-type alpha1A et de 2 pour le sous-type alpha1B. On en conclut que le dextromilnacipran est un antagoniste pour ces deux sous-types de récepteurs alpha1-adrénergiques. Le lévomilnacipran est aussi un antagoniste des récepteurs alpha1A et alpha1B, mais avec une puissance beaucoup plus faible que celle du dextromilnacipran.

Exemple 3

Caractéristiques pharmacologiques des mélanges de lévomilnacipran et dextromilnacipran, en faisant varier les proportions des énantiomères

Objectifs

Le lévomilnacipran, (énantiomère, 1S,2R) a un profil pharmacologique distinct, en regard du racémique milnacipran (2207) et l'autre énantiomère 1R,2S (dextromilnacipran). Le lévomilnacipran est l'énantiomère le plus actif sur les cibles désirées : la liaison au transporteur noradrénaline (NET), la liaison au transporteur sérotonine (SERT) et le site PCP (récepteur NMDA, système du glutamate), mais est le moins actif sur les cibles non-désirées: c'est-à-dire sur les récepteurs adrénergiques $\alpha$1A et $\alpha$1B. Nous avons estimé les propriétés pharmacologiques de différents mélanges (contenant des pourcentages variés de dextromilnacipran). Premièrement, des expériences de liaison ont été simulées et les constantes d'inhibitions apparentes des mélanges pour NET, SERT et les récepteurs $\alpha$1A ont été calculées. Deuxièmement, la simulation était expérimentalement validée pour les récepteurs $\alpha$1A, pour lesquels les variations de lévomilnacipran avaient le plus d'impact.

Méthodes

Simulation

Les constantes d'inhibition (valeur de $K_i$) du lévomilnacipran et du dextromilnacipran sur les principales cibles ont été déterminées expérimentalement par des expériences de liaisons classiques utilisant des radioligands spécifiques et des protéines recombinantes humaines (voir exemple 1).
Pour chaque cible, le total de radioligand lié a été calculé en prenant en compte l'effet inhibiteur de chaque inhibiteur. Le total de chaque cible occupée est décrit par la loi d'action de masse classique :

$$(1) \quad \frac{R.S}{B} = K_d$$

$$(2) \quad \frac{R.I_1}{RI_1} = K_{i1}$$

$$(3) \quad \frac{R.I_2}{RI_2} = K_{i2}$$

$$(4) \quad Bmax = B + RI_1 + RI_2 + R$$

où R est la concentration de sites de liaison libre ; Bmax, la concentration totale de sites ; B, la concentration de sites liés au radioligand ; S, la concentration de radioligand ; $K_d$, la constante de dissociation du radioligand ; $i_1$, la concentration de l'inhibiteur 1 ; $K_{i1}$, la constante d'inhibition de l'inhibiteur 1 ; $RI_1$, la concentration de sites occupés par l'inhibiteur 1 ; $i_2$, la concentration de l'inhibiteur 2 ; $Ki_2$, la constante d'inhibition de l'inhibiteur 2 et RI2 est la concentration de sites occupés par l'inhibiteur 2.

A partir des équations (1) à (4) :

$$B = \frac{Bmax \cdot S}{S + K_d (1 + i_1/K_{i1} + I_2/K_{i2})}$$

Les valeurs de B étaient calculées en tenant compte des valeurs actuelles de $K_i 1$ et $K_i 2$ (voir tableau ci-dessus), en faisant varier de 0,01% à 30% la proportion de dextromilnacipran dans le mélange, et en faisant varier la concentration du mélange de 0,01 à 52,0 $\mu$M. Les valeurs de $K_d$ et S qui étaient utilisées étaient similaires aux valeurs des expériences de liaison. En conséquence, une courbe d'inhibition théorique était dessinée avec chaque combinaison des valeurs des différents paramètres. Puis, la valeur d'$IC_{50}$ apparente pour chaque courbe était calculée par une régression non-linéaire selon l'équation logistique :

$$Y = 100/1 + 10(i - LogIC_{50})$$

où i est la concentration de l'inhibiteur (mélange), et la valeur de $K_i$ apparente était dérivée par l'équation de Cheng-Prussoff: $IC_{50} = K_i (1 + S/K_d)$.

2.2 Expériences de liaison

Une série de mélange de lévomilnacipran et dextromilnacipran, en faisant varier les proportions de dextromilnacipran, était préparée et chaque mélange était incubé à des concentrations variées avec des membranes de cellules exprimant le récepteur recombinant humain $\alpha$1A et de la prazosine[3H] comme radioligand. La valeur d'$IC_{50}$ apparente pour chaque courbe était calculée par une régression non-linéaire selon l'équation logistique :

$$Y = 100/1 + 10(i - LogIC_{50}),$$

où i est la concentration de l'inhibiteur (mélange), et la valeur de $K_i$ apparente était dérivée par l'équation de Cheng-Prussoff: $IC_{50} = K_i (1 + S/K_d)$.

Résultats

Les résultats sont exprimés comme valeur de $K_i$ apparente, en fonction du pourcentage de dextromilnacipran (Figure 1).

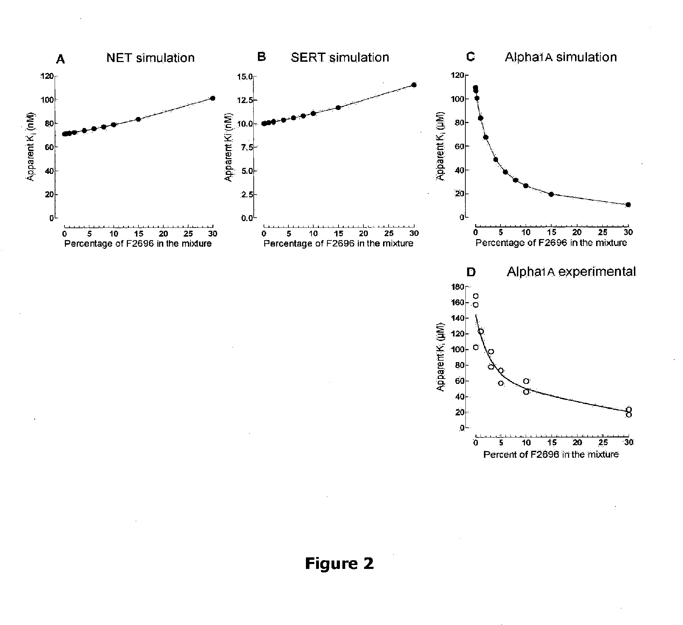

La figure 2 montre : les valeurs de $K_i$ apparentes des expériences simulées (A, B et C) et mesurées (D) des mélanges de lévomilnacipran (F2695) et dextromilnacipran (F2696) avec des proportions de dextromilnacipran croissante pour les cibles NET, SERT ou les récepteurs $\alpha$1A.

Les valeurs de $K_i$ pour NET et SERT n'étaient pas trop touchées par différentes proportions de dextromilnacipran. Au contraire, la valeur de $K_i$ apparente pour les récepteurs $\alpha$1A, soit des expériences de simulation ou des expériences réelles, était dramatiquement touchée quand le pourcentage de dextromilnacipran augmentait, ce qui indique que l'impact sur les récepteurs $\alpha$1 n'est pas négligeable. Si on considère que l'impact dévient non négligeable quand la valeur de Ki chute de moitié, le pourcentage maximal de dextromilnacipran est d'environ 5%.

Conclusions

Un mélange avec une proportion de dextromilnacipran supérieure à ces 5% peut ne pas être bioéquivalent au lévomilnacipran "substantiellement pur", ou au mélange avec des proportions plus faibles de dextromilnacipran. L'impact sur les récepteurs $\alpha$1A, des mélanges avec des proportions de dextromilnacipran supérieures à 5% n'est pas négligeable et de tels mélanges ne devraient pas être utilisés en traitement dans la récupération fonctionnelle après AVC.

**Revendications**

1. Mélange lévomilnacipran / dextromilnacipran contenant du dextromilnacipran dans une proportion ne dépassant pas 5 % en masse dudit mélange pour son utilisation comme médicament pour la récupération et la réhabilitation fonctionnelle après accident neurologique aigu et ses récidives.

2. Le mélange selon la revendication 1 pour son utilisation chez des patients ayant reçu le diagnostic d'accident vasculaire cérébral, d'origine ischémique ou hémorragique

3. Le mélange selon la revendication 1 pour son utilisation chez des patients ayant reçu le diagnostic de traumatisme crânien.

4. Compositions pharmaceutiques comprenant au moins un excipient pharmaceutiquement acceptable et un mélange lévomilnacipran / dextromilnacipran contenant du dextromilnacipran dans une proportion ne dépassant pas 5 % en masse dudit mélange à titre de principe actif, pour son utilisation en tant que médicament dans la récupération et la réhabilitation fonctionnelle après accident neurologique aigu et ses récidives.

5. Compositions pharmaceutiques pour son utilisation selon la revendication 4 chez des patients ayant reçu le diagnostic d'accident vasculaire cérébral.

6. Compositions pharmaceutiques pour son utilisation selon la revendication 4 chez des patients ayant reçu le diagnostic de traumatisme crânien.

7. Compositions pharmaceutiques pour son utilisation selon l'une quelconque des revendications 4 à 6, **caractérisées en ce que** le dosage quotidien du lévomilnacipran est compris entre 50 et 200 mg.

8. Compositions pharmaceutiques pour son utilisation selon l'une des revendications 4 à 7, **caractérisées en ce qu'**elles se présentent sous une forme à absorption intestinale modifiée permettant l'administration d'une seule dose par jour.

**Patentansprüche**

1. Levomilnacipran/Dextromilnacipran-Gemisch, das Dextromilnacipran in einem Verhältnis enthält, das 5 Ma% des Gemischs nicht überschreitet, für seine Nutzung als Arzneimittel für die Rekonvaleszenz und funktionelle Rehabilitation nach einem akuten neurologischen Ereignis und seinen Rezidiven.

2. Das Gemisch nach Anspruch 1 für seine Verwendung bei Patienten, welche die Diagnose eines zerebralen vaskulären Ereignisses ischämischer oder hämorrhagischer Ursache erhalten haben.

3. Das Gemisch nach Anspruch 1 für seine Verwendung bei Patienten, welche die Diagnose eines Schädeltraumas erhalten haben.

4. Pharmazeutische Zusammensetzungen, die mindestens einen pharmazeutisch akzeptablen Hilfsstoff und ein Levomilnacipran/Dextromilnacipran-Gemisch, das Dextromilnacipran in einem Verhältnis enthält, das 5 Ma% des Gemischs nicht überschreitet, als Wirkstoff für seine Nutzung als Arzneimittel in der Rekonvaleszenz und funktionellen Rehabilitation nach einem akuten neurologischen Ereignis und seinen Rezidiven.

5. Pharmazeutische Zusammensetzungen für seine Nutzung nach Anspruch 4 bei Patienten, welche die Diagnose eines zerebralen vaskulären Ereignisses erhalten haben.

6. Pharmazeutische Zusammensetzungen für seine Nutzung nach Anspruch 4 bei Patienten, welche die Diagnose eines Schädeltraumas erhalten haben.

7. Pharmazeutische Zusammensetzungen für seine Nutzung nach einem der Ansprüche 4 bis 6, **dadurch gekennzeichnet, dass** die tägliche Dosierung des Levomilnacipran zwischen 50 und 200 mg inklusive ist.

8. Pharmazeutische Zusammensetzungen für seine Nutzung nach einem der Ansprüche 4 bis 7, **dadurch gekennzeichnet, dass** sie sich in einer Form mit modifizierter Darmabsorption präsentieren, welche die Verabreichung einer einzigen Dosis pro Tag erlaubt.


**Claims**

1. A levomilnacipran/dextromilnacipran mixture containing dextromilnacipran in a proportion not exceeding 5% by weight of said mixture for use as medicinal product for recovery and functional rehabilitation after an acute neurological event and recurrences thereof.

2. The mixture according to claim 1 for use in patients diagnosed with cerebral vascular accident of ischemic or hemorrhagic origin.

3. The mixture according to claim 1 for use in patients diagnosed with traumatic brain injury.

4. Pharmaceutical compositions comprising at least one pharmaceutically acceptable excipient and a levomilnacipran/dextromilnacipran mixture containing dextromilnacipran in a proportion not exceeding 5% by weight of said mixture as active ingredient for use as medicinal product for recovery and functional rehabilitation after an acute neurological event and recurrences thereof.

5. Pharmaceutical compositions for use according to claim 4 in patients diagnosed with cerebrovascular accident.

6. Pharmaceutical compositions for use according to claim 4 in patients diagnosed with traumatic brain injury.

7. Pharmaceutical compositions for use according to any of claims 4 to 6, **characterized in that** the daily dosage of levomilnacipran is between 50 and 200 mg.

8. Pharmaceutical compositions for use according to one of claims 4 to 7, **characterized in that** they are in a modified intestinal absorption form allowing the administration of a single dose per day.

**Figure 1**

**Figure 2**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2004075886 A
- WO 2009127737 A
- WO 2003039598 A
- WO 2003068211 A
- WO 2003077897 A
- WO 2003090743 A
- WO 2004009069 A
- WO 2004030633 A
- WO 2004045718 A
- WO 2007038620 A
- WO 2008019388 A
- WO 2008021932 A
- WO 2008147843 A
- WO 2006006617 A
- EP 939626 A

**Littérature non-brevet citée dans la description**

- **MURRAY CJ ; LOPEZ AD.** Mortality by cause for eight régions of the world: Global Burden of Disease Study. *Lancet,* 1997, vol. 349, 1269-1276
- **GOLDSTEIN LB.** Basic and clinical studies of pharmacological effects on recovery from brain injury. *J neural Transplant & Plasticity,* 1993, vol. 4, 175-192
- **FEENEY DM ; DE SMET AM ; RAI S.** Noradrenergic modulation of hemiplegia: facilitation and maintenance of recovery. *Restor Neurol & Neurosci,* 2004, vol. 22, 175-190
- **SONDE L ; NORDSTRÖM M ; NILSSON CG ; LÖKK J ; VIITANEN M.** A double-blind placebo-controlled study of the effects of amphetamine and physiotherapy after stroke. *Cerebrovasc Dis,* 2001, vol. 12, 253-257
- **NISHINO K ; SASAKI T ; TAKAHASHI K ; CHIBA M ; ITO T.** The norepinephrine precursor L-threo-3,4-dihydroxyphenylserine facilitates motor recovery in chronic stroke patients. *J Clin Neurosci,* 2001, vol. 8, 547-550
- **TARDY J ; PARIENTE J ; LEGER A ; DECHAUMONT-PALACIN S ; GERDELAT A ; GUIRAUD V ; CONCHOU F ; ALBUCHER JF ; MARQUE P ; FRANCERIES X.** Methylphenidate modulates cerebral post-stroke reorganization. *Neuroimage,* 2006, vol. 33, 913-922
- **ZITTEL S ; WEILLER C ; LIEPERT J.** Reboxetine improves motor function in chronic stroke. A pilot study. *J Neurol,* 2007, vol. 254, 197-201
- **PARIENTE J ; LOUBINOUX I ; CAREL C ; ALBUCHER JF ; LEGER A ; MANELFE C ; RASCOL O ; CHOLLET F.** Fluoxetine modulates motor performance and cerebral activation of patients recovering from stroke. *Ann Neurol,* 2001, vol. 50, 718-729

- **CHOLLET F ; TARDY J ; ALBUCHER JF ; THALAMAS C ; BERARD E ; LAMY C ; BEJOT Y ; DELTOUR S ; JAILLARD A ; NICLOT P.** Fluoxetine for motor recovery after acute ischaemic stroke (FLAME): a randomised placebo-controlled trial. *Lancet Neurol,* 2011, vol. 10, 123-30
- **BRILEY M ; PROST JF ; MORET C.** Preclinical pharmacology of milnacipran. *Int Clin Psychopharmacol,* 1996, vol. 4, 9-14
- **PRESKORN SH.** Milnacipran: a dual norepinephrine and serotonin reuptake pump inhibitor. *J Psychiatr Pract,* 2004, vol. 10, 119-26
- **SPENCER CM ; WILDE MI.** Milnacipran : a review of its use in depression. *Drugs,* 1998, vol. 56, 405-427
- **OWEN RT.** Milnacipran hydrochloride: its efficacy, safety and tolerability profile in fibromyalgia syndrome. *Drugs Today (Barc),* 2008, vol. 44, 653-60
- Catecholamines, sympathomimetic drugs and adrenergic receptor antagonists. **HOFFMAN ; LEFKOWITZ.** Goodman and Gilman's The Pharmacological Basis of Therapeutics. McGraw-Hill, 1995, 229
- **FEENEY DM ; WESTERBERG VS.** Norepinephrine and brain damage: alpha noradrenergic pharmacology alters functional recovery after cortical trauma. *Can J Psychology,* 1990, vol. 44, 233-252
- **STIBICK DL ; FENNEC DM.** Enduring vulnerability to transient reinstatement of hemiplegia by prazosin after traumatic brain injury. *J Neurotrauma,* 2001, vol. 18, 303-312
- **SAWAKI L ; WERHAHN KJ ; BARCO R ; KOPYLEV L ; COHEN LG.** Effect of an alpha1-adrenergic blocker on plasticity elicited by motor training. *Exp Brain Res,* 2003, vol. 148, 504-508

- **GOLDSTEIN et al.** The influence of drugs on the recovery of sensorimotor function after stroke. *J Neuro Rehab,* 1990, vol. 4, 137-144
- **BERGE SM ; BIGHLEY LD ; MONKHOUSE DC.** *Pharmaceutical salts,* 1977, vol. 66, 1-19
- **WURCH T ; BOUTET-ROBINET EA ; PALMIER C ; COLPAERT FC ; PAUWELS PJ.** Constitutive coupling of chimeric dopamine D2/alpha1B receptor to the phospholipase C pathway : inverse agonism to silent antagonism of neuroleptic drugs. *J Pharmacol Exp Ther,* 2003, vol. 304, 380-390
- **VICENTIC et al.** Biochemistry and pharmacology of epitope-tagged alpha1a-adrenergic receptor subtype. *J Pharm Exp Ther,* 2002, vol. 302, 58-65